# FASCICULE DE BREVET EUROPEEN

(11) **EP 3 487 984 B1**
(45) Date de publication et mention de la délivrance du brevet: **24.08.2022**
(21) Numéro de dépôt: 17748551.3
(22) Date de dépôt: 19.07.2017
(51) Int. Cl.: C12N 1/18, A21D 8/04, C12R 1/865

(54) **LEVURES DE PANIFICATION OPTIMISÉES**
VERBESSERTE HEFEN FÜR DIE BROTHERSTELLUNG
ENHANCED BREAD-MAKING YEASTS

(30) Priorité: 20.07.2016 FR 1656902
(43) Date de publication de la demande: 29.05.2019
(73) Titulaire: Lesaffre et Compagnie, 75001 Paris (FR)
(72) Inventeur: BARTOLUCCI, Jean-Charles, 59560 Hellemmes-Lille (FR); FONCHY-PENOT, Evelyne, 62840 Fleurbaix (FR); IMBERT-PODGORSKI, Jennifer, 59370 Mons en Baroeul (FR); KAPRAL, Florence, 59560 Comines (FR); PARASIE, Georges, 59320 Sequedin (FR); QUIPOURT-ISNARD, Anne-Dominique, 59700 Marc En Baroeul (FR); TRIONE, Valérie, 59560 Comines (FR)
(74) Mandataire: Plasseraud IP
(86) Numéro de dépôt international: PCT/FR2017/051970
(87) Numéro de publication internationale: WO 2018/015660

(56) Documents cités:
- EP-A2- 1 338 647
- WO-A1-2015/092208
- A. TEUNISSEN ET AL: "Isolation and Characterization of a Freeze-Tolerant Diploid Derivative of an Industrial Baker's Yeast Strain and Its Use in Frozen Doughs", APPLIED AND ENVIRONMENTAL MICROBIOLOGY, vol. 68, no. 10, 1 octobre 2002 (2002-10-01), pages 4780-4787, XP055309217, ISSN: 0099-2240, DOI: 10.1128/AEM.68.10.4780-4787.2002

## Description

### Domaine technique de l'invention

La présente invention concerne des souches de levure de panification également appelées levures de boulangerie. De telles souches trouvent particulièrement un intérêt dans la production de produits panaires. La présente invention concerne particulièrement une souche de levure déposée le 19 mai 2016 auprès de la CNCM sous le No. I-5088.

### Arrière-plan technologique

La production de produits de panification à l'échelle industrielle nécessite l'utilisation de levures dites de boulangerie. De telles levures doivent permettre une fermentation efficace à grande échelle pour différents procédés de panification.

Le procédé de type « Sponge and Dough » est un procédé de panification très largement utilisé dans le milieu industriel. Durant ce procédé, la panification est pratiquée avec deux étapes de fermentation : une première étape « SPONGE » qui correspond à une première fermentation d'une pâte comprenant plus de la moitié de la farine totale mise en œuvre, une partie de l'eau et tout ou partie de la levure, et une seconde étape de fermentation « DOUGH », dans laquelle le SPONGE est combiné avec le reste de la farine, le reste de l'eau et les autres ingrédients de la pâte. Un tel procédé de panification nécessite l'utilisation de souches ayant des capacités de fermentation élevées permettant notamment d'obtenir un fort dégagement gazeux (CO2) particulièrement durant la phase Dough.

Le document WO 2015/092208 décrit un procédé de mutagenèse et de sélection d'une souche de levure de panification ayant une force fermentative augmentée.

A ce jour, la souche de référence utilisée pour ce type de fermentation est la souche correspondant à la levure commercialisée sous la référence Red Star Cream Yeast, désignée ci-après par RSCY qui présente de bonnes capacités de fermentation et qui est compatible avec une utilisation industrielle. Néanmoins, dans un schéma d'utilisation industrielle, il serait très intéressant de :
- développer des levures présentant des capacités de fermentation encore plus élevées ; et/ou
- de diminuer la quantité de levures mises en œuvre ; et/ou
- de diminuer le temps nécessaire à la fermentation de la pâte.

### Description détaillée de l'invention

Dans ce cadre, les présents inventeurs ont développé plusieurs souches de levures présentant des capacités de fermentation améliorées par rapport à la souche de référence *RSCY.*

Les levures développées dans le cadre de la présente invention ont été obtenues par mutagénèse de la souche de référence RSCY, et sélectionnées car elles permettent d'augmenter les dégagements gazeux d'environ 10% dans le Dough par rapport à la souche de référence RSCY, et/ou de de diminuer de 10% la quantité de matière sèche de levure utilisée et/ou de réduire de 10% le temps nécessaire pour la fermentation.

Les inventeurs ont particulièrement développé la souche de levure déposée le 19 mai 2016 auprès de la CNCM (Collection Nationale de Cultures de Micro-organismes, 25/28 rue du docteur ROUX, F-75724 Paris Cedex 15) en vertu du traité de Budapest sous le No. I-5088. Cette souche présente une force fermentative sur schéma Sponge and Dough nettement plus élevée que celle enregistrée avec la souche de référence, avec notamment une augmentation de dégagement gazeux de 9-10% par rapport à la souche *RSCY et* ce dans un laps de temps 5 à 12 % inférieur.

Ainsi, dans un premier aspect, la présente invention concerne une souche de levure qui est la souche déposée le 19 mai 2016 auprès de la CNCM en vertu du traité de Budapest sous le numéro I-5088.

L'expression « souche de levure » désigne une population relativement homogène de cellules de levure. Une souche de levure est obtenue à partir d'un clone, un clone étant une population de cellules obtenue à partir d'une seule cellule de levure.

Les souches de levures ainsi modifiées sont particulièrement intéressantes car elles peuvent être utilisées dans un procédé de panification à l'échelle industrielle.

La présente invention concerne également une levure issue d'une souche de levure telle que décrite ci-dessus.

La souche de levure selon la présente invention a été obtenue par un procédé comprenant une étape de mutagénèse de la souche de référence RSCY suivie d'une étape de sélection des mutants ainsi obtenus selon leur capacités fermentatives.

La mutagénèse consiste à introduire des mutations dans le matériel génétique de la levure en exposant cette dernière à des agents mutagènes. Un exemple d'agent mutagène qui peut être utilisé dans le cadre de la présente invention est le rayonnement UV.

Le programme de mutagénèse a donné lieu à l'obtention de très nombreux mutants, pratiquement 10 000 mutants. Ceux-ci ont intégré un processus de sélection complexe comprenant plusieurs niveaux d'évaluation toujours basés sur la comparaison du pouvoir ferment de ces mutants par rapport à la souche de référence.

Finalement ont été retenus les mutants ayant le meilleur dégagement de CO2 en application Sponge and Dough mais aussi en application en schéma de panification direct.

Les levures utilisées pour la panification peuvent être sous plusieurs formes. Par exemple, la levure selon la présente invention peut être sous forme de crème de levure, de levure pressée, de levure sèche ou de levure surgelée.

Les levures fraîches sont caractérisées par une teneur élevée en eau par rapport aux levures sèches. Les levures fraîches englobent les crèmes de levure et les levures pressées.

Les crèmes de levure, appelées également « levures liquides », sont des suspensions aqueuses de cellules de levure présentant une viscosité de type crème. Ces suspensions aqueuses de cellules vivantes de levure, ont généralement une teneur en matière sèche d'au moins 12% en poids, particulièrement de 12 à 40 % en poids. Une crème de levure peut avoir par exemple une teneur en matière sèche comprise entre 12 et 25% en poids, de préférence entre 14 et 22% en poids.

Les levures pressées comprennent les levures pressées en bloc compact ainsi que les levures pressées émiettées. Les levures pressées en bloc compact, appelées également « pains de levure », sont caractérisées par une teneur en matière sèche comprise entre 26% et 35%. Les levures pressées émiettées présentent elles une teneur en eau comprise entre 21% et 35%.

Les levures sèches sont caractérisées par une teneur en matière sèche supérieure à environ 92%.

Les levures surgelées sont caractérisées par une teneur en matière sèche comprise entre 74% et 80%.

L'invention concerne également l'utilisation d'une souche de levure telle que décrite ci-dessus pour la production de produits de panification.

Dans un mode de réalisation particulier, la levure selon la présente invention est sous forme de crème de levure.

La panification regroupe l'ensemble des opérations consistant à transformer la farine en pain.

Les levures selon l'invention peuvent être utilisées dans des procédés de panification de type schéma direct (« NO-TIME DOUGH ») ou de type schéma indirect (« SPONGE and DOUGH »).

Un schéma direct ne comporte pratiquement pas de première fermentation entre un pétrissage intensif et la division de la pâte, les pâtons obtenus étant fermentés en moule entre 35°C et 40°C, puis cuits.

Les levures selon la présente invention sont particulièrement performantes dans les procédés de panification, en particulier dans un procédé de panification de type Sponge and Dough. Ce type de procédé est décrit par exemple dans le livre de référence « Bakers Handbook » de E. J. Pyler, publié par Sosland Publishing Co.

Ainsi, selon un autre aspect, la présente invention concerne l'utilisation d'une souche de levure telle que décrite ci-dessus dans un procédé de panification de type Sponge and Dough.

Un schéma « SPONGE and DOUGH » est un procédé de panification comprenant deux étapes de fermentation :
- une première étape, appelée « SPONGE », qui correspond à la fermentation d'une pâte comprenant 50 à 70% de la farine totale mise en œuvre, une partie de l'eau et tout ou partie de la levure, pendant plusieurs heures, en général entre 3 et 6 heures, et plus particulièrement entre 3 et 4 heures ; et
- une seconde étape, appelée « DOUGH », dans laquelle le SPONGE obtenu après la fermentation ci-dessus est combiné avec le reste de la farine, le reste de l'eau et les autres ingrédients de la pâte, le mélange ainsi constitué est pétri, divisé, mis en moule et fermenté, puis cuit, cette seconde fermentation en moule correspondant à l'apprêt, sa durée étant le temps d'apprêt.

Les pourcentages sont exprimés en pourcentages dits du boulanger, le pourcentage dit du boulanger étant une méthode de calcul appliquée aux rapports des ingrédients dans laquelle la masse totale de la farine représente toujours 100% et la masse des autres ingrédients de la pâte est calculée par rapport à cette masse de farine.

La présente invention concerne donc un procédé de panification de type Sponge and Dough comprenant une étape de fermentation par une levure selon l'invention.

Selon un mode de réalisation particulier, l'invention vise un procédé de panification, particulièrement de type Sponge and Dough comprenant une étape de fermentation par une levure selon l'invention caractérisée en ce que le temps d'apprêt de la pâte boulangère est inférieur de 8%, particulièrement 10%, encore plus particulièrement 12% par rapport au temps nécessaire pour la fermentation de ladite pâte boulangère par la souche de référence RSCY.

Selon un autre mode de réalisation particulier, l'invention vise un procédé de panification, particulièrement de type Sponge and Dough, comprenant une étape de fermentation par une levure selon l'invention caractérisé en ce que la quantité de levure nécessaire pour la fermentation de la pâte boulangère est inférieure de 10% par rapport à la quantité de levure nécessaire pour la fermentation de ladite pâte boulangère avec la souche de référence RSCY.

L'invention vise encore une pâte boulangère contenant une levure selon l'invention. Particulièrement, la présente invention vise une pâte boulangère comprenant des levures dont au moins 10% sont des levures telles que décrites ci-dessus. Dans un mode de réalisation particulier, la pâte boulangère selon la présente invention comprend des levures dont au moins 25%, 50% ou 75% sont des levures telles que décrites ci-dessus.

La pâte boulangère peut être une pâte sans sucre, une pâte légèrement sucrée ou une pâte sucrée, le sucre étant le plus souvent du sirop de sucre ou du saccharose.

L'expression « pâte légèrement sucrée » désigne des pâtes ayant une teneur en sucre ajouté inférieure à 14% en masse par rapport à la masse de la farine, de préférence inférieure ou égale à 12% en masse par rapport à la masse de la farine.

L'invention vise encore un procédé de préparation d'une pâte boulangère comprenant une étape de fermentation par une levure selon l'invention.

Selon un mode de réalisation particulier, le procédé de préparation de pâte boulangère selon la présente invention est de type Sponge and Dough.

### EXEMPLES

### Obtention de levures présentant des capacités fermentatives élevées

Un programme de mutagenèse par bombardement UV a été réalisé sur la souche de référence RSCY, qui a donné lieu à l'obtention de nombreux mutants. Ceux-ci ont intégré un processus de sélection complexe comprenant plusieurs niveaux d'évaluation toujours basés sur la comparaison du pouvoir ferment de ces souches par rapport à la souche de référence RSCY. Ainsi ce processus a permis la sélection la souche I-5088 parmi les 10000 mutants évalués.

La souche I-5088 issue de ce processus de sélection a été produite en fermenteur 20L et évaluée en panification Sponge and Dough. Deux campagnes d'essais ont été menées. Pour chaque campagne d'essai, 3 cuves sont réalisées avec la souche I-5088 de manière indépendante. On procède de même avec la souche de référence RSCY.

La formule de panification sponge and dough employée est indiquée dans le tableau suivant. Cette formule correspond à la situation où toute la levure est mise dans le sponge. Les quantités sont exprimées en pourcentages du boulanger, à savoir rapportées à la quantité de farine mise en œuvre. La dose de levure est ajustée selon la matière sèche mesurée pour chaque crème de levure, de manière à toujours obtenir une quantité de 1.275% de matière sèche levure en base farine dans le pétrin. La quantité d'eau de coulage dans le sponge est ajustée en fonction de la quantité de levure de manière à ce que le total des deux soit toujours égal à 42.5%.

| **Ingrédient** | **% dans le sponge** | **% dans le dough** | **% total** |
|---|---|---|---|
| Farine | 70 | 30 | 100 |
| Eau | 35.5 | 16 | 51.5 |
| Levure Liquide à 18.2% | 7 | - | 7 |
| Yeast Food | 0.25 | - | 0.25 |
| Sodium stearoyl lactylate | 0.5 | - | 0.5 |
| Alpha-amylase | 0.0002 | - | 0.0002 |
| High Fructose Corn Syrup | - | 15 | 15 |
| Sel | - | 1.8 | 1.8 |
| Propionate de calcium | - | 0.5 | 0.5 |
| Huile de soja | - | 3 | 3 |
| Monoglycérides | - | 1 | 1 |
| Gluten Vital | - | 1 | 1 |
| Améliorant Lesoft | - | 0.25 | 0.25 |

Le diagramme de panification suivi a été le suivant pour la préparation du sponge :
- La double enveloppe des pétrins Hobart est tempérée avec une eau à 22°C
- L'eau de coulage utilisée est à 24°C +/- 1°C
- La température de pâte visée est de 25°C
- Le pétrissage a lieu dans des pétrins Hobart équipés de cuves Mac Duffy, selon le diagramme suivant :
   ∘ Les ingrédients secs sont prémélangés pendant 1 minute
   ∘ Les ingrédients liquides sont ensuite incorporés
   ∘ 1 minute en vitesse 1
   ∘ 2 minutes en vitesse 2
- A l'issue du pétrissage, les pâtes sont décuvées, installées dans des culs de poule qui sont placés dans une enceinte régulée à 30°C et 90% HRE pendant 4 heures. Il s'agit d'une fermentation en masse.

Le diagramme de panification suivi a été le suivant pour la préparation du dough :
- On procède à un nouveau pétrissage comme suit :
   ∘ Les ingrédients du dough sont placés dans le pétrin
   ∘ 1 minute en vitesse 1
   ∘ Incorporation du sponge
   ∘ 1 minute en vitesse 1
   ∘ 3 minutes en vitesse 2.
- A la sortie du pétrissage le temps de pointage en masse est de 5 minutes, à l'issue duquel les pâtes sont divisées (3 pièces de 516g par pétrin) et boulées manuellement.
- Après une détente de 5 minutes, les pâtons sont façonnés dans une façonneuse horizontale et les pâtons façonnés sont placés dans des moules lesquels sont placés dans une enceinte régulée à 43°C et 90% d'Humidité Relative (HRE).
- A l'issue de cette phase de fermentation finale, que l'on appelle l'apprêt, on procède à la cuisson pendant 23 minutes dans un four à balancelle Reed, préchauffé à 180°C.
- Les pains sont ensuite sortis du four et ressuent une heure.

Les mesures qui sont réalisées pour quantifier l'activité fermentaire sont de trois types :
1. A l'issue du pétrissage des doughs, on prélève 50g de chaque pétrissée, que l'on place dans un pot destiné à la mesure de la production de gaz via un Risograph^{®}. On exploite le cumul de gaz après deux heures (DG2H).
2. Lors de l'introduction des moules dans l'enceinte régulée à 43°C, on place des gabarits sur chaque moule, dont le niveau dépasse de 1.2 cm le haut du moule. On mesure le temps nécessaire à la pâte présente dans le moule pour atteindre cette hauteur. Ce temps est dénommé le Proof Time (PT), il est mesuré en minutes.
3. Pour certains pâtons, on procède à une cuisson après un temps constant. On mesure le volume et la masse des pains issus de cette cuisson. Le rapport du volume par la masse nous permet d'estimer le volume spécifique (VS) de chaque pain, après un temps constant de fermentation.

Les résultats obtenus avec les crèmes produites à partir de la souche I-5088 sont rapportés à ceux obtenus avec la souche de référence RSCY. Dans le tableau suivant les résultats sont donc exprimés en pourcentage d'écart.

| | % d'écart moyen entre 3 crèmes produites avec I-5088 et 3 crèmes produites avec RSCY | | |
|---|---|---|---|
| | % DG2H | % PT | % VS |
| Campagne d'essai N°1 | +9% | -5% | 0% |
| Campagne d'essai N°2 | +11% | -8% | +1% |

Ces résultats démontrent que la souche I-5088 permet d'obtenir un gain de Proof Time de 5 à 8% et un gain de dégagement gazeux de 9-11% par rapport aux crèmes réalisées dans les mêmes conditions avec la souche de référence RSCY.

## Revendications

1. Souche de levure déposée le 19 mai 2016 auprès de la Collection Nationale de Cultures de Micro-organismes (CNCM) en vertu du traité de Budapest sous le No. I-5088.

2. Levure obtenue par culture de la souche de levure selon la revendication 1.

3. Levure selon la revendication 2, **caractérisée en ce qu'**elle se présente sous une forme de crème de levure.

4. Utilisation d'une levure selon l'une des revendications 2 à 3 dans un procédé de panification.

5. Utilisation selon la revendication 4 **caractérisée en ce que** ledit procédé de panification est de type Sponge and Dough.

6. Utilisation selon la revendication 5, **caractérisée en ce que** ledit procédé nécessite une quantité de levure inférieure de 10% par rapport à la quantité de levure nécessaire lorsque la souche de référence RSCY est utilisée.

7. Pâte boulangère obtenue par utilisation d'une levure selon l'une des revendications 4 à 6 dans un procédé de panification.

## Patentansprüche

1. Hefestamm, der am 19. Mai 2016 bei der Collection Nationale de Cultures de Microorganismes (CNCM) nach dem Budapester Vertrag unter der Nr. 1-5088 hinterlegt wurde.

2. Hefe, die durch Kultivierung des Hefestamms nach Anspruch 1 erhalten wird.

3. Hefe nach Anspruch 2, **dadurch gekennzeichnet, dass** sie in Form einer Hefecreme vorliegt.

4. Verwendung einer Hefe nach einem der Ansprüche 2 bis 3 in einem Brotbackverfahren.

5. Verwendung nach Anspruch 4, **dadurch gekennzeichnet, dass** das Brotbackverfahren vom Typ _{"}Sponge and Dough" ist.

6. Verwendung nach Anspruch 5, **dadurch gekennzeichnet, dass** das Verfahren eine um 10% geringere Hefemenge erfordert, bezogen auf die Hefemenge, die erforderlich ist, wenn der Referenzstamm RSCY verwendet wird.

7. Backteig, der durch Verwendung einer Hefe nach einem der Ansprüche 4 bis 6 in einem Brotbackverfahren erhalten wird.

## Claims

1. A yeast strain deposited on May 19, 2016, at the Collection Nationale de Cultures de Microorganismes (CNCM) pursuant to the Budapest treaty under No. 1-5088.

2. A yeast obtained by culturing the yeast strain according to claim 1.

3. The yeast as claimed in claim 2, **characterized in that** it is in a cream yeast form.

4. Use of a yeast as claimed in one of claims 2 to 3, in a bread-making process.

5. The use as claimed in claim 4, **characterized in that** said bread-making process is of Sponge and Dough type.

6. The use as claimed in claim 5, **characterized in that** said process requires an amount of yeast of less than 10% as compared to the amount of yeast required when the RSCY reference strain is used.

7. A baking dough obtained by using a yeast as claimed in one of claims 4 to 6 in a bread-making process.
